(19) Europäisches Patentamt
European Patent Office
Office européen des brevets

(11) **EP 2 130 497 A1**

(12) **EUROPEAN PATENT APPLICATION**

(43) Date of publication:
**09.12.2009 Bulletin 2009/50**

(51) Int Cl.:
*A61B 8/08* (2006.01)    *G06T 5/00* (2006.01)
*G06T 7/00* (2006.01)

(21) Application number: **09161527.8**

(22) Date of filing: **29.05.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **05.06.2008  KR 20080053231**

(71) Applicants:
• **MEDISON CO., LTD.**
**Gangwon-do 250-875 (KR)**
• **Korea Advanced Institute of Science and Technology**
**Daejeon 305-338 (KR)**

(72) Inventors:
• **Hyun, Dong Gyu**
**135-851, Seoul (KR)**

• **Ra, Jong Beom**
**30-762, Daejeon (KR)**
• **Nam, Woo Hyun**
**616-806, Busan (KR)**
• **Kang, Dong-Goo**
**441-400, yeonggi-do (KR)**
• **Lee, Duhgoon**
**302-170, Daejeon (KR)**

(74) Representative: **Schmid, Wolfgang**
**Lorenz & Kollegen**
**Alte Ulmer Strasse 2**
**89522 Heidenheim (DE)**

(54) **Anatomical feature extraction from an ultrasound liver image**

(57)    There is disclosed an embodiment for extracting anatomical features from a 3-dimensional B-mode ultrasound liver image for image registration. A system extracts anatomical features from a 3-dimensional B-mode ultrasound liver image for image registration. An image forming unit forms a 3-dimensional ultrasound liver image based on ultrasound signals reflected from the liver. A diaphragm extracting unit extracts a diaphragm region from the 3-dimensional B-mode ultrasound liver image. A vessel extracting unit extracts vessel regions from the 3-dimensional ultrasound liver image. A diaphragm refining unit refines the extracted diaphragm region with the extracted vessel regions to remove clutters from the diaphragm region. A registration unit extracts sample points from the diaphragm region with the clutters removed and the vessel regions for image registration.

FIG. 1

**Description**

The present application claims priority from Korean Patent Application No. 10-2008-0053231 filed on June 5, 2008, the entire subject matter of which is incorporated herein by reference.

TECHNICAL FIELD

The present disclosure relates to anatomical feature extractions, and more particularly to an anatomical feature extraction from a 3-dimensional B-mode ultrasound liver image.

BACKGROUND

An ultrasound diagnostic system has been extensively used in the medical field due to its non-invasive and non-destructive nature. The ultrasound diagnostic system is highly safe and has no dangerous side effects such as exposure to X-rays, etc. However, the ultrasound diagnostic system suffers from inherent shortcomings of an ultrasound image such as a low signal-to-noise ratio and a limited field of view. Thus, the image registration of a CT (or MR) image onto the ultrasound image has been introduced in order to compensate for deficiencies of the ultrasound image.
Generally, a 3-dimensional ultrasound liver image may include obstacles to deter anatomical feature extractions such as speckle noises, mirroring image artifacts, shading artifacts or appearance of other organs in a region of interest. These obstacles may cause a registration error during the ultrasound-CT image registration.

SUMMARY

Embodiments for extracting anatomical features from a 3-dimensional ultrasound liver image are disclosed herein. In one embodiment, by way of non-limiting example, a system for extracting anatomical features from an ultrasound image, comprises: an image forming unit configured to form a 3-dimensional ultrasound liver image based on ultrasound signals reflected from a liver; a diaphragm extracting unit configured to extract a diaphragm region from the 3-dimensional ultrasound liver image; a vessel extracting unit configured to extract vessel regions from the 3-dimensional ultrasound liver image; and a diaphragm refining unit configured to remove clutters from the diaphragm region based on the extracted vessel regions to thereby refine the extracted diaphragm region.
In another embodiment, a method of extracting anatomical features from an ultrasound image, comprises: a) forming a 3-dimensional ultrasound liver image based on ultrasound signals reflected from a liver; b) extracting a diaphragm region from the 3-dimensional ultrasound liver image; c) extracting vessel regions from the 3-dimensional ultrasound liver image; d) removing clutters from the diaphragm region based on the extracted vessel regions to thereby refine the extracted diaphragm region; and e) extracting sample points from the diaphragm region with the clutters removed and the vessel regions.
The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

BRIEF DESCRIPTION OF THE DRAWINGS

FIG. 1          is a block diagram showing an illustrative embodiment of a system for extracting anatomical features from a 3-dimensional B-mode ultrasound liver image.
FIG. 2A         shows an example of an ultrasound liver image before removing speckle noises.
FIG. 2B         shows an example of an ultrasound liver image with speckle noises removed.
FIG. 3          is a schematic diagram showing an example of eigenvalues in the Hessian matrix.
FIGS. 4A to 4D  show examples of images formed through diaphragm extraction.
FIG. 5A         shows an example of an image before ROI masking.
FIG. 5B         shows an example of an image with ROI masked.
FIG. 6          shows an example of images obtained through vessel extraction in one embodiment.
FIG. 7A         shows an example of images resulting from flatness test, morphological filtering, size test and refinement for data A and B with noises removed.
FIG. 7B         shows an example of images resulting from segmentation of vessel candidates, initial vesselness test and final vesselness test for data A and B with speckle noises removed.

## DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

In one embodiment, an anatomical feature extraction from an ultrasound image of a target object for image registration, e.g., image registration between an ultrasound image and a computerized tomography (CT) image, will be described. In one embodiment, the target object may be a liver, although it is certainly not limited thereto. Also, the ultrasound image of the target object may be a 3-dimensional brightness (B)-mode liver image.

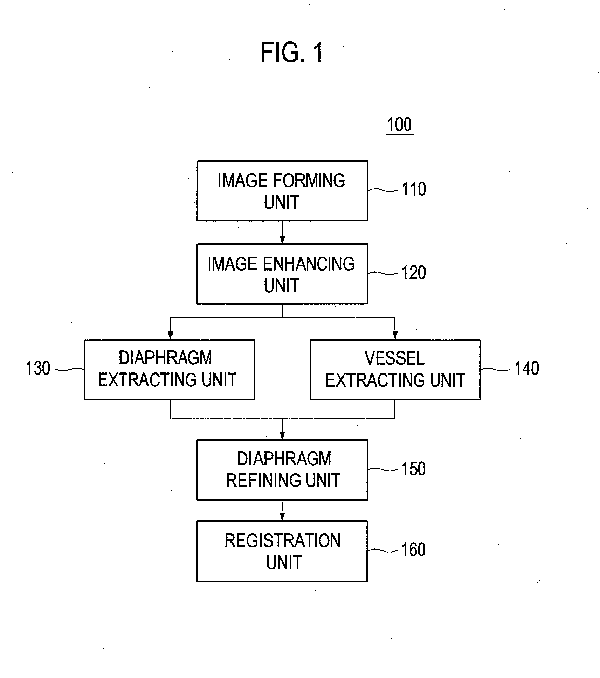

As depicted in FIG. 1, a system 100 may include an image forming unit 110. The image forming unit may be configured to transmit/receive ultrasound signals to/from the target object (e.g., liver) to thereby output electrical receive signals. The image forming unit 110 may be further configured to form a 3-dimensional ultrasound image of the target object. Hereinafter, the 3-dimensional ultrasound image of the target object will be referred to as a 3-dimensional B-mode ultrasound liver image.

The system 100 may further include an image enhancing unit 120. The image enhancing unit 120 may be configured to receive observed signals for forming the 3-dimensional B-mode ultrasound liver image from the image forming unit 110. The image enhancing unit 120 may be further configured to perform speckle noise filtering to remove speckle noises contained in the observed signals. The image enhancing unit 120 may be also configured to increase an edge contrast to thereby enhance anatomical feature definition in the 3-dimensional B-mode ultrasound liver image with the speckle noise removed. In one embodiment, the speckle noise filtering may be carried out by applying the total variation based speckle constraint filtering algorithm in combination with an anisotropic diffusion to the 3-dimensional B-mode ultrasound liver image. This speckle noise filtering may be performed such as the following equations (1) to (5).

The observed signals $u_0$ containing the speckle noises may be modeled as the following equation (1).

$$u_0 = u + \sqrt{u}\,n \qquad\qquad (1)$$

wherein $n$ represents a zero-mean Gaussian variable having standard deviation and $u$ represents original signals not containing the speckle noises.
There is a constraint in the relationship between the observed signals $u_0$ and the original signal $u$ according to the speckle noise model of the equation (1) as the following equation (2).

$$\frac{1}{|\Omega|}\int_{\Omega}\frac{(u-u_0)^2}{u}d\Omega = \sigma_n^2 \qquad\qquad (2)$$

wherein $\Omega$ represents a dimension of the image and $\sigma_n$ represents the standard deviation. The following equation (3) represents the total variation of the original signals $u$.

$$\int_{\Omega}\|\nabla u\|d\Omega \qquad\qquad (3)$$

If the equation (3) is minimized with the constraint condition of the equation (2), then the speckle noises may be removed to thereby obtain image signals close to the original signals $u$.
The following equation (4) may be derived from the equations (2) and (3) by the constraint Euler-Lagrange equation.

$$\frac{\partial u}{\partial t} = div(F) - \lambda(t)\left(\frac{u^2 - u_0^2}{u}\right), in\,\Omega \qquad\qquad (4)$$

wherein $\lambda(t)$ represents an attachment coefficient for preventing that the image signals with the speckle noises removed steeply differ from the observed signals according to iteration.

It is important to appropriately determine the attachment coefficient $\lambda(t)$. In order to determine the attachment coefficient $\lambda(t)$, $(u - u_0)u/(u + u_0)$ is multiplied to both sides of the equation (4) and an integral may then be performed over an entire range of $\Omega$.

In a Steady state, a left side of the equation (4) will be converged to $0$ so that the following equation (5) may be easily derived.

$$\lambda(t) = \frac{1}{\sigma_n^2 |\Omega|} \int (\frac{u - u_0}{u + u_0} u) div(F) d\Omega \qquad (5)$$

wherein a new value of $\lambda(t)$ is iteratively obtained. The equation (4) may be solved by applying these values, iteratively, under the assumption that $\partial u/\partial t$ is $0$. This is so that image signals with the speckle noises removed, i.e., image signals $u$ close to the original signals, may be obtained, iteratively. For example, the solution of the equation (4) may be iteratively carried out about 40 times so that desirable image signals with the speckle noises removed may be obtained.

Further, if the speckle noise filtering is performed without considering the orientations of the edges as above, then degradation of the sharpness of the edges may occur. Thus, the speckle noise filtering may be carried out in consideration with the orientations of the edges for preventing the degradation of the edge sharpness, i.e., while the sharpness of edges neighboring to feature points is maintained. An anisotropic diffusion function F for reflecting the orientation may be defined as the following equation (6).

$$F = D\nabla u \qquad (6)$$

wherein D represents a diffusion matrix.

As mentioned above, the image signals close to the original signals can be obtained through the above speckle noise filtering. Also, since the speckle noises are removed mainly in a tangential direction to the edges due to the characteristic of the anisotropic diffusion rather than in a normal direction to the edges, gradients may be enhanced to the normal direction of the edges. Thus, the edges may be more sharpened. That is, the speckle noises may be removed while increasing sharpness of the feature points.

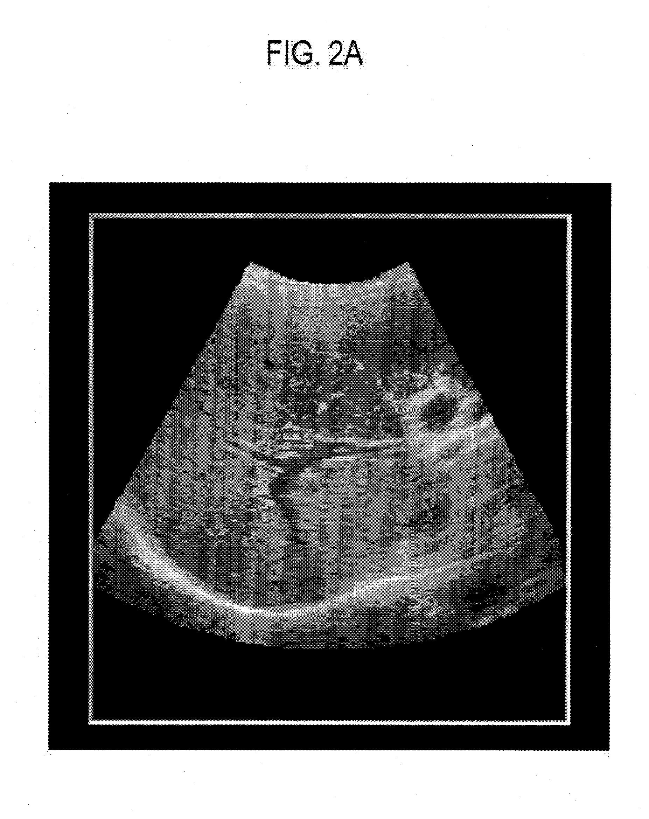

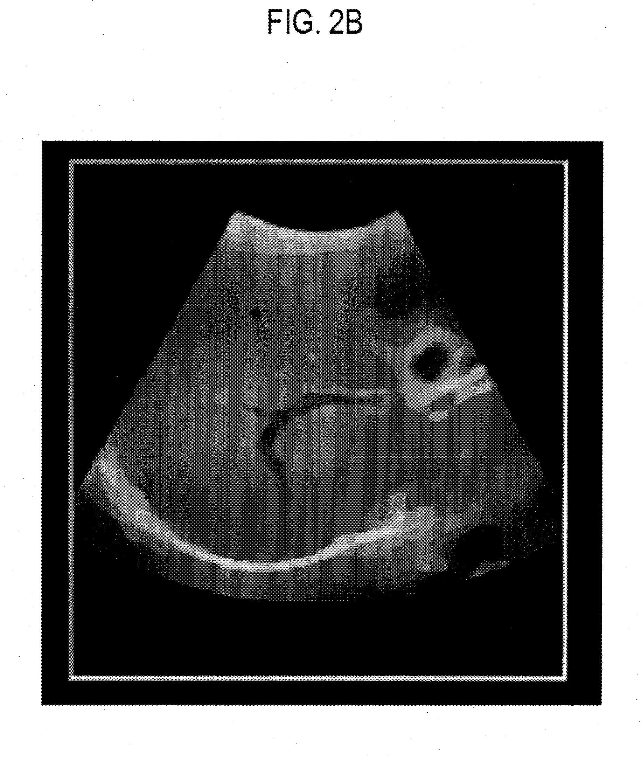

FIG. 2A shows an example of an ultrasound liver image before removing the speckle noises, while FIG. 2B shows an example of an ultrasound liver image with the speckle noises removed.

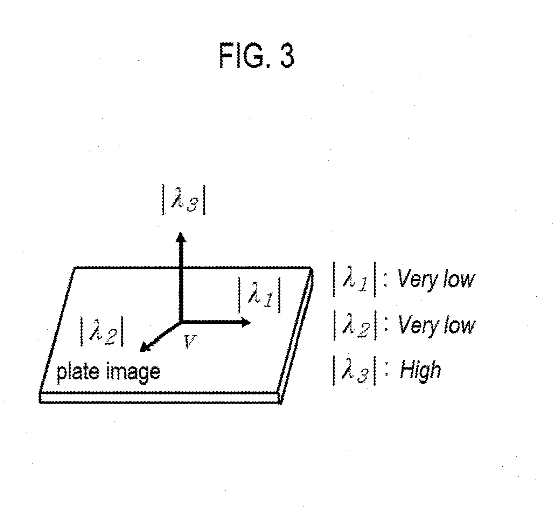

The system 100 may further include a diaphragm extracting unit 130. The diaphragm extracting unit may be configured to extract a diaphragm from the 3-dimensional B-mode ultrasound liver image with the edge contrast enhanced. In one embodiment, the diaphragm may perform a Hessian matrix based flatness test upon the 3-dimensional B-mode ultrasound liver image to extract the diaphragm. The diaphragm may be considered as a curved surface in the 3-dimensional ultrasound liver image. Thus, regions in which a voxel intensity change in a normal direction at a surface is greater than a voxel intensity change in a horizontal direction at the surface may be extracted as the diaphragm. FIG. 3 is a schematic diagram showing an example of eigenvalues $\lambda_1$, $\lambda_2$ and $\lambda_3$ in the Hessian matrix.

Hereinafter, an operation of the diaphragm extracting unit 130 will described in detail. The diaphragm extracting unit 130 may be configured to select voxels having a relatively high flatness value. In a 2-dimensional B-mode ultrasound liver image, the voxles may be represented with pixels. The flatness $\mu(v)$ may be defined as the following equation (7).

$$\mu(v) = \phi_1(v)\phi_2(v)\phi_3(v)/\phi_{3_{max}}(v) \qquad (7)$$

$\Phi_1(v)$, $\Phi_2(v)$ and $\Phi_3(v)$ in the equation (7) may be represented as the following equation (8).

$$\phi_1(v) = \left(1 - \frac{\lambda_1(v)}{\lambda_3(v)}\right)^2, \ \phi_2(v) = \left(1 - \frac{\lambda_2(v)}{\lambda_3(v)}\right)^2, \ \phi_3(v) = \sum_i \lambda_i(v)^2 \qquad (8)$$

wherein $\lambda_1$, $\lambda_2$ and $\lambda_3$ denote eigenvalues of the Hessian matrix at voxel *v*. The flatness $\mu(v)$ may be normalized to have values of ranging 0-1. A flatness map may be formed based on the flatness values obtained from all of the voxels according to the equations (6) and (7). Thereafter, the voxles having a relatively high flatness value are selected. In one embodiment, the voxels having the flatness over 0.1 may be selected.

The diaphragm extracting unit 130 may be further configured to perform the morphological opening upon the selected voxels to remove small clutters therefrom. The morphological opening may be carried out by sequentially performing erosion and dilation. That is, morphological boundaries in which the voxel values exist are removed as many as the predetermined number of the voxels and then contracted (erosion). Thereafter, the boundaries are expanded as many as the predetermined number of the voxels. In one embodiment, the boundaries may be contracted and expanded by 1 voxel.

The diaphragm is the largest surface in the 3-dimensional B-mode ultrasound liver image. The largest surface may be selected among candidate surfaces obtained by the intensity-based connected component analysis (CCA) for the voxles and the selected surface may be regarded as the diaphragm in the ultrasound liver image. The voxel-based CCA is one of the methods of grouping regions in which voxel values exist.

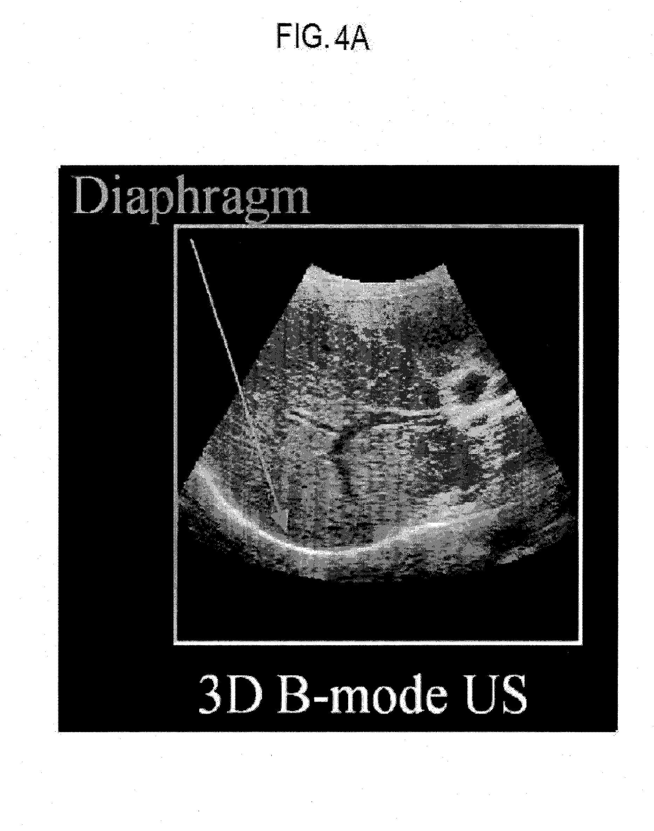

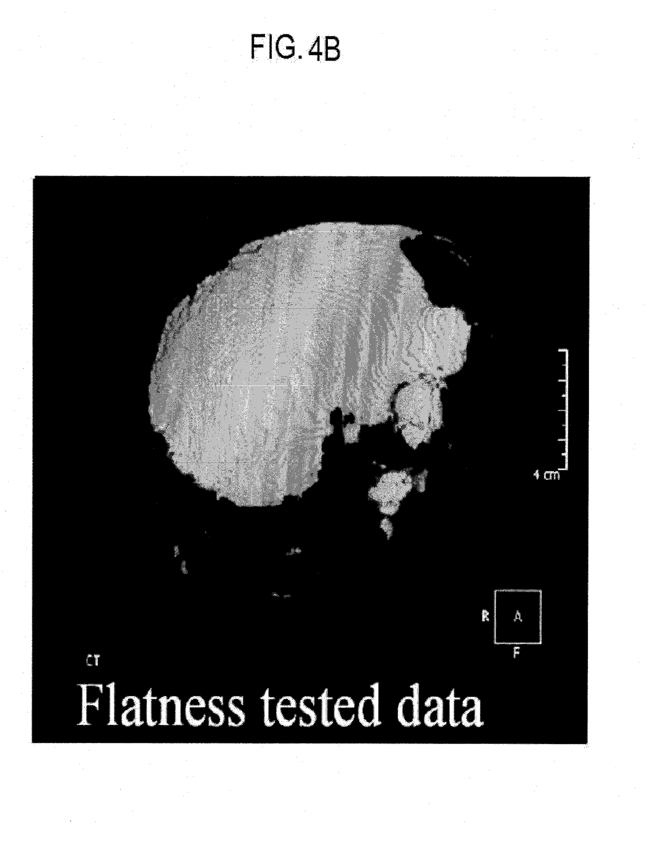

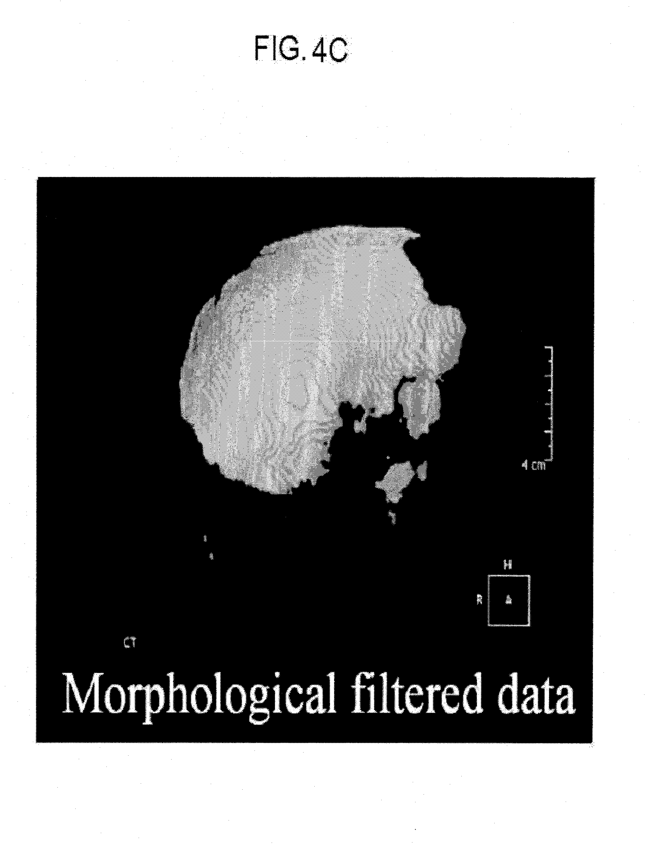

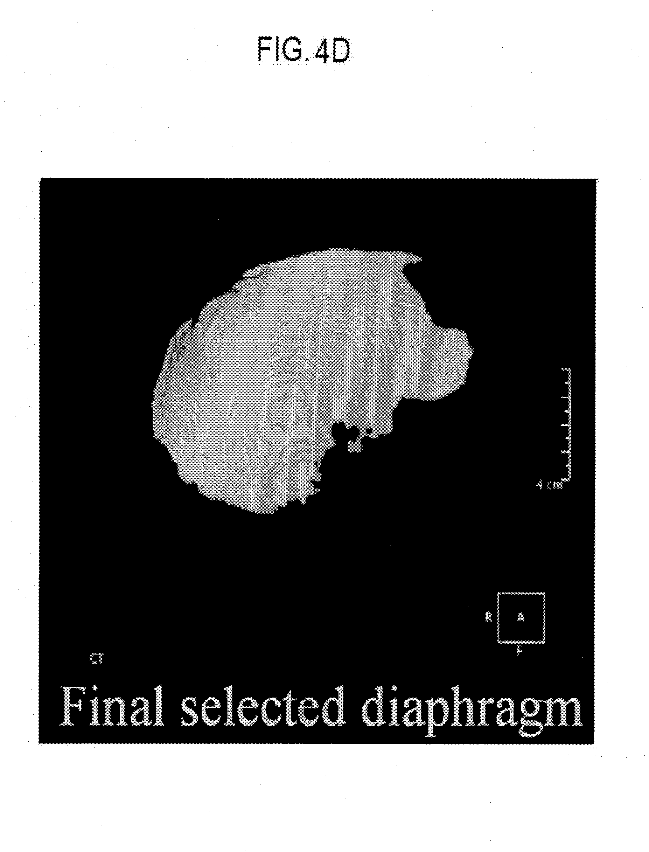

For example, the number of voxels connected to each of the voxels through a connectivity test by referring to values of voxels neighboring to the corresponding voxel (e.g., 26 voxels) may be computed. The voxels, of which connected voxels are greater than the predetermined number, are selected as candidate groups. Since the diaphragm is the widest curved surface in the ROI, the candidate group having the most connected voxels may be selected as the diaphragm. The surface of the diaphragm may be smoothened. FIGS. 4A to 4D show examples of images formed through diaphragm extraction. FIG. 4A shows an example of an ultrasound image with the diaphragm indicated, while FIG. 4B shows an example of an image obtained through the flatness test. Also, FIG. 4C shows an example of an image obtained through morphological filtering, while FIG. 4D shows an example of a final selected diaphragm image.

The system 100 may further include a vessel extracting unit 140 that may operable to perform vessel extraction upon the 3-dimensional B-mode ultrasound liver image with the edge contrast enhanced. The vessel extracting unit 140 may be configured to perform the vessel extraction through ROI masking, vessel segmentation and classification.

To avoid mis-extraction of the vessels due to mirroring artifacts, the ROI masking may be applied to the 3-dimensional B-mode ultrasound liver image with the edge contrast enhanced by modeling the diaphragm as a polynomial curved surface. In such a case, the ROI masking, which models the diaphragm as the polynomial curved surface by using the least means square, may be used. However, if all of the lower portions of the modeled polynomial curved surface are eliminated, meaningful vessel information may be lost at a portion of regions due to an error of the polynomial curved surface.

To avoid losing the vessel information, the lower portion of the modeled polynomial curved surface may be eliminated with a marginal distance. For example, the marginal distance may be set to about 10 vessels at a lower portion of the ROI mask.

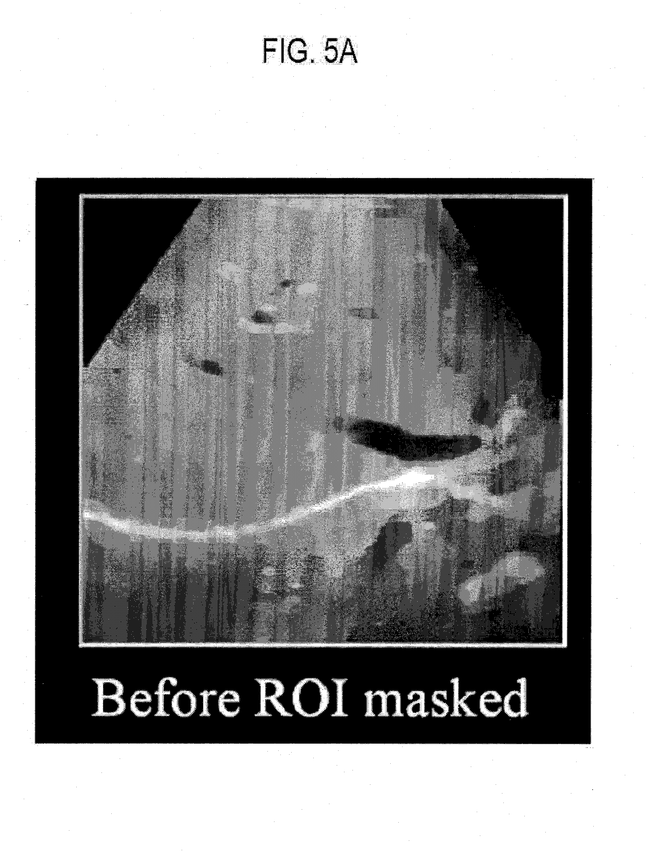

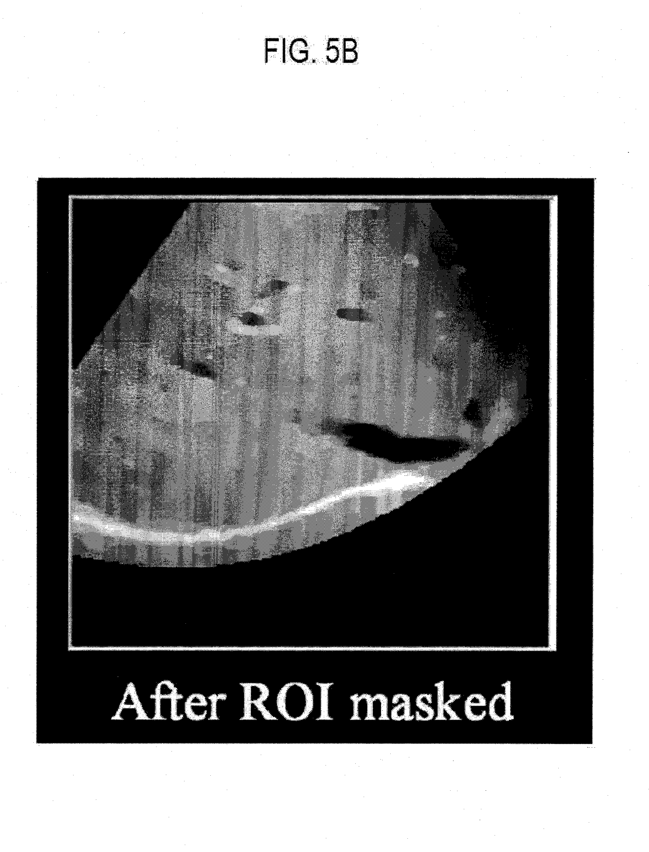

FIG. 5A shows an example of an image before ROI masking, while FIG. 5B shows an example of an image with ROI masked.

Subsequently, the vessel extracting unit 140 may be further configured to segment a vessel region and a non-vessel region. To exclude non-vessel high intensity regions such as the diaphragm and vessel walls, a low intensity bound having a less reference bound value in the ROI masked image may be estimated. Thereafter, voxels with a higher intensity value than the reference bound value may be removed. An adaptive threshold scheme may be applied to the remaining regions for binarization thereof.

The binary segments may be labeled as vessel candidates.

Next, the vessel extracting unit 140 may be further configured to remove non-vessel-type clutters from the binarization image to classify real vessels from vessel candidates. In one embodiment, the vessel classification may include a size test, which evaluates the goodness of fit to a cylindrical tube, for filtering out tiny background clutters, a structure-based vessel test for removing non-vessel type clutters, i.e., an initial vessel test, gradient magnitude analysis, and a final vessel test for perfectly removing the clutters. Although some clutters are not removed through the structure-based vessel test, an initial threshold may be marginally set so that all vessels may be included.

For example, the initial threshold may be set to 0.6. At the final vessel test, clutters, which may be formed by small shading artifacts having low gradient magnitudes, may be perfectly removed by considering variation of voxel values, i.e., gradient magnitudes, to thereby extract vessel data. In one embodiment, a threshold of the final vessel test may be set to 0.4.

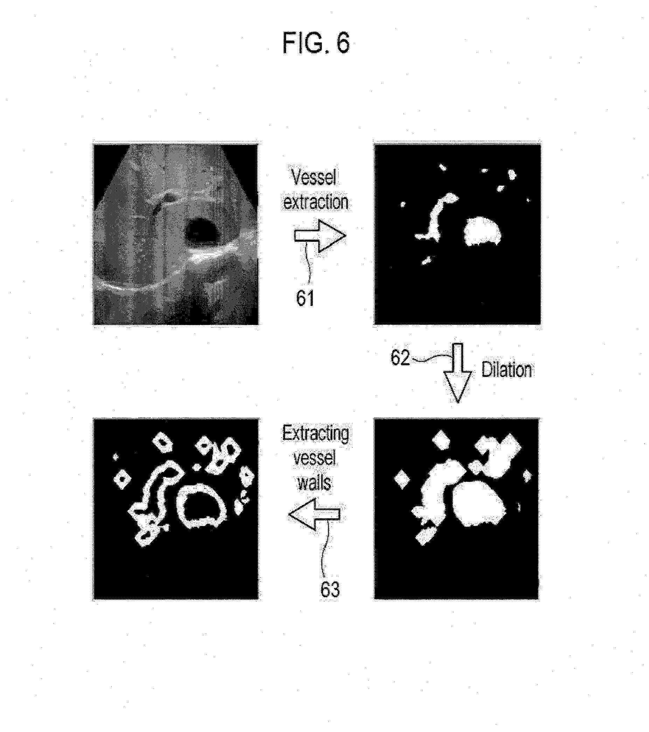

The system 100 may further include a diaphragm refining unit 150 that may be operable to refine the diaphragm region by removing the clutters with the extracted vessel data. The clutters are mainly placed near the vessel walls. Especially, the vessel wall of inferior vena cava (IVC) is more likely to be connected to the diaphragm and cause clutters. These clutters may degrade the accuracy of feature based registration so that the diaphragm should be refined. To refine the diaphragm, the vessel region is extracted according to the vessel extraction mentioned above, the extracted vessel region may be dilated, and then the dilated vessel region may be subtracted from the initially extracted diaphragm region to estimate vessel walls. The estimated vessel walls may be removed from the diaphragm region. FIG. 6 shows an example of images obtained through the vessel extraction. As shown in FIG. 6, the vessel regions are extracted at arrow 61 and the extracted vessel regions are dilated at arrow 62. Then, the vessel walls are extracted from the dilated image at arrow 63. Finally, the diaphragm region may be extracted by applying CCA and the size test.

Further, the system 100 may include a registration unit 160 that may be operable to perform the image registration between the 3-dimensional ultrasound and CT liver images. The registration unit 160 may extract sample points from the vessel region and the diaphragm region, respectively, among the features extracted from the 3-dimensional B-mode ultrasound image. Also, after the vessel region and the diaphragm region are extracted from the CT image, the registration unit 160 may be operable to extract sample points from the vessel and the diaphragm region, respectively. In one embodiment, the ICP based registration may be performed with the extracted sample points from the respective ultrasound and CT images for registration.

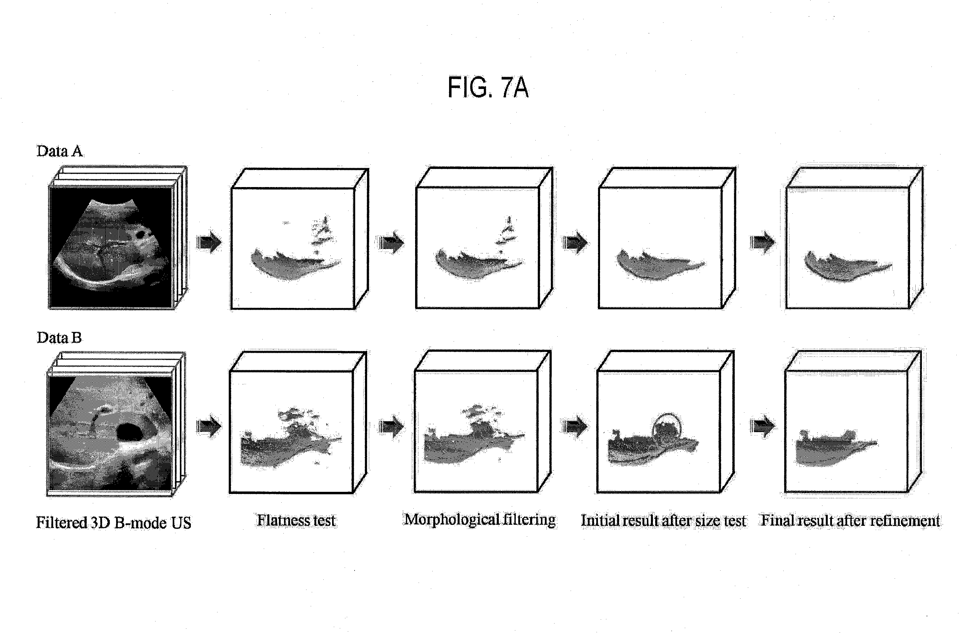

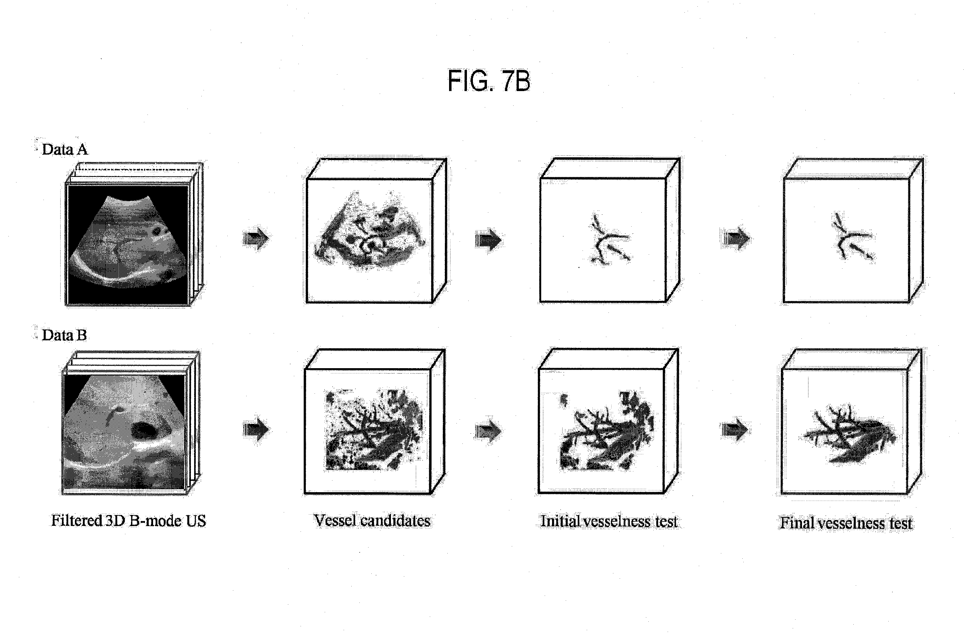

FIG. 7A shows an example of images resulting from flatness test, morphological filtering, size test and refinement for the 3-dimensional B-mode ultrasound images A and B (Data A and B) with speckle noises removed. In such a case, Data A having a size of 200*200*200 and isotropic voxel resolution of 0.8, and Data B having a size of 200*188*168 and isotropic voxel resolution of 0.6, are experimentally used. FIG. 7B shows an example of images resulting from segmentation of vessel candidates, initial vesselness test and final vesselness test for the 3-dimensional B-mode ultrasound images A and B (Data A and B) with speckle noises removed.

As mentioned above, since the image registration is performed by using anatomical features of the 3-dimensional ultrasound liver, the image registration error between the ultrasound-CT images may be reduced.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

**Claims**

1. A system for extracting anatomical features from an ultrasound image, comprising:

   an image forming unit configured to form a 3-dimensional ultrasound liver image based on ultrasound signals reflected from a liver;
   a diaphragm extracting unit configured to extract a diaphragm region from the 3-dimensional ultrasound liver image;
   a vessel extracting unit configured to extract vessel regions from the 3-dimensional ultrasound liver image; and
   a diaphragm refining unit configured to remove clutters from the diaphragm region based on the extracted vessel regions to thereby refine the extracted diaphragm region.

2. The system of Claim 1, wherein the 3-dimensional ultrasound image is a 3-dimensional B-mode ultrasound liver image.

3. The system of Claim 2, further comprising an image enhancing unit configured to remove speckle noises from the 3-dimensional ultrasound image and enhance a contrast of the 3-dimensional ultrasound image with the speckle noises removed.

4. The system of Claim 3, wherein the image enhancing unit is further configured to apply total variation based speckle constraint filtering algorithm in combination with an anisotropic diffusion to the 3-dimensional ultrasound liver image to remove the speckle noises.

5. The system of Claim 3, wherein the image enhancing unit is further configured to perform speckle noise filtering mainly in a tangential direction of edges in the 3-dimensional B-mode liver image rather than the normal direction to the edges.

6. The system of Claim 2, wherein the diaphragm extracting unit is further configured to:

   extract the diaphragm from the 3-dimensional B-mode ultrasound liver image;
   obtain flatness from voxels of the 3-dimensional B-mode ultrasound liver image with the speckle noises removed to from a flatness map;

select voxels having a flatness greater than a reference value;

remove edges, in which the voxel values exist, as many as the predetermined number of the voxels, contract the voxels and expand the edges as many as the predetermined number of the voxels to thereby remove first clutters;

obtain candidate surfaces from the voxels with the first clutters removed by the intensity-based connected component analysis (CCA); and

select a widest surface among the candidate surfaces to extract the diaphragm region.

7. The system of Claim 6, wherein the vessel extracting unit is further configured to:

extract the vessel regions from the 3-dimensional B-mode ultrasound image;

model the diaphragm region to a polynomial curved surface in the 3-dimensional B-mode ultrasound image for region of interest (ROI) masking, wherein the ROI masking is performed to remove a lower portion of the modeled polynomial curved surface with a marginal distance;

remove voxels having a intensity value greater than a reference bound value to select vessel candidates; and

remove non-vessel-type clutters from the selected vessel candidates to classify real vessels.

8. The system of Claim 7, wherein the vessel extracting unit is further configured to perform a structure-based vessel test for removing non-vessel type clutters, gradient magnitude analysis, and a final vessel test for perfectly removing the clutters.

9. A method of extracting anatomical features from an ultrasound image, comprising:

a) forming a 3-dimensional B-mode ultrasound liver image based on ultrasound signals reflected from a liver;

b) extracting a diaphragm region from the 3-dimensional B-mode ultrasound liver image;

c) extracting vessel regions from the 3-dimensional B-mode ultrasound liver image;

d) removing clutters from the diaphragm region based on the extracted vessel regions to thereby refine the extracted diaphragm region; and

e) extracting sample points from the diaphragm region with the clutters removed and the vessel regions.

10. The method of Claim 9, further comprising removing speckle noises from the 3-dimensional ultrasound image and enhancing a contrast of the 3-dimensional ultrasound image with the speckle noises removed.

11. The method of Claim 10, further comprising applying total variation based speckle constraint filtering algorithm in combination with an anisotropic diffusion to the 3-dimensional ultrasound liver image to thereby remove the speckle noises.

12. The method of Claim 10, further comprising performing speckle noise filtering mainly in a tangential direction of edges in the 3-dimensional B-mode liver image rather than the normal direction to the edges.

13. The method of Claim 9, further comprising:

extracting the diaphragm from the 3-dimensional B-mode ultrasound liver image;

obtaining flatness from voxels of the 3-dimensional B-mode ultrasound liver image with the speckle noises removed to from a flatness map;

selecting voxels having a flatness greater than a reference value;

removing edges, in which the voxel values exist, as many as the predetermined number of the voxels, contracting the voxels and expand the edges as many as the predetermined number of the voxels to thereby remove first clutters;

obtaining candidate surfaces from the voxels with the first clutters removed by the intensity-based connected component analysis (CCA); and

selecting a widest surface among the candidate surfaces to extract the diaphragm region.

14. The method of Claim 13, further comprising:

extracting the vessel regions from the 3-dimensional B-mode ultrasound image;

modeling the diaphragm region to a polynomial curved surface in the 3-dimensional B-mode ultrasound image for region of interest (ROI) masking, wherein the ROI masking is performed to remove a lower portion of the modeled polynomial curved surface with a marginal distance;
removing voxels having a intensity value greater than a reference bound value to select vessel candidates; and
removing non-vessel-type clutters from the selected vessel candidates to classify real vessels.

15. The method of Claim 14, further comprising performing a structure-based vessel test for removing non-vessel type clutters, gradient magnitude analysis, and a final vessel test for perfectly removing the clutters.

# FIG. 1

100

```
┌─────────────────────┐
│   IMAGE FORMING     │ ～ 110
│        UNIT         │
└─────────────────────┘
          │
          ▼
┌─────────────────────┐
│  IMAGE ENHANCING    │ ～ 120
│        UNIT         │
└─────────────────────┘
     │           │
     ▼           ▼
┌──────────────┐ ┌──────────────┐
│  DIAPHRAGM   │ │    VESSEL    │ ～ 140
│ EXTRACTING   │ │  EXTRACTING  │
│    UNIT      │ │    UNIT      │
└──────────────┘ └──────────────┘
  130 ～
     │           │
     ▼
┌─────────────────────┐
│     DIAPHRAGM       │ ～ 150
│   REFINING UNIT     │
└─────────────────────┘
          │
          ▼
┌─────────────────────┐
│    REGISTRATION     │ ～ 160
│        UNIT         │
└─────────────────────┘
```

# FIG. 2A

## FIG. 2B

# FIG. 3

$|\lambda_1|$ : Very low

$|\lambda_2|$ : Very low

$|\lambda_3|$ : High

# FIG.4A

# FIG. 4B

## FIG. 4C

Morphological filtered data

# FIG. 4D

CT

Final selected diaphragm

# FIG. 5A

Before ROI masked

## FIG. 5B

After ROI masked

# FIG. 6

# FIG. 7A

Data A

Data B

Filtered 3D B-mode US | Flatness test | Morphological filtering | Initial result after size test | Final result after refinement

EP 2 130 497 A1

# FIG. 7B

Data A

Data B

Filtered 3D B-mode US          Vessel candidates          Initial vesselness test          Final vesselness test

EP 2 130 497 A1

EP 2 130 497 A1

Europäisches Patentamt
European Patent Office
Office européen des brevets

## EUROPEAN SEARCH REPORT

Application Number

EP 09 16 1527

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 0 991 015 A (KONINKL PHILIPS ELECTRONICS NV [NL]) 5 April 2000 (2000-04-05) * paragraph [0002] * * paragraph [0004] - paragraph [0016] * ----- | 1-15 | INV. A61B8/08 G06T5/00 G06T7/00 |
| A | US 2003/236460 A1 (MA QINGLIN [US] ET AL) 25 December 2003 (2003-12-25) * paragraph [0002] - paragraph [0008] * * paragraph [0041] - paragraph [0052] * ----- | 1-15 | |
| A | US 2007/167784 A1 (SHEKHAR RAJ [US] ET AL) 19 July 2007 (2007-07-19) * paragraph [0051] - paragraph [0073] * * paragraph [0121] - paragraph [0140] * ----- | 1-15 | |
| | | | TECHNICAL FIELDS SEARCHED (IPC) A61B G06T |

The present search report has been drawn up for all claims

| Place of search | Date of completion of the search | Examiner |
|---|---|---|
| Munich | 14 October 2009 | Montes, Pau |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

22

**ANNEX TO THE EUROPEAN SEARCH REPORT
ON EUROPEAN PATENT APPLICATION NO.**

EP 09 16 1527

This annex lists the patent family members relating to the patent documents cited in the above-mentioned European search report.
The members are as contained in the European Patent Office EDP file on
The European Patent Office is in no way liable for these particulars which are merely given for the purpose of information.

14-10-2009

| Patent document cited in search report | | Publication date | Patent family member(s) | | Publication date |
|---|---|---|---|---|---|
| EP 0991015 | A | 05-04-2000 | DE<br>JP<br>US | 69922317 D1<br>2000102538 A<br>6190320 B1 | 05-01-2005<br>11-04-2000<br>20-02-2001 |
| US 2003236460 | A1 | 25-12-2003 | NONE | | |
| US 2007167784 | A1 | 19-07-2007 | NONE | | |

For more details about this annex : see Official Journal of the European Patent Office, No. 12/82

**EP 2 130 497 A1**

**REFERENCES CITED IN THE DESCRIPTION**

**Patent documents cited in the description**

- KR 1020080053231